(19) Europäisches Patentamt
European Patent Office
Office européen des brevets

(11) EP 3 862 022 B1

(12) **EUROPEAN PATENT SPECIFICATION**

(45) Date of publication and mention of the grant of the patent:
**12.06.2024 Bulletin 2024/24**

(21) Application number: **19869672.6**

(22) Date of filing: **02.10.2019**

(51) International Patent Classification (IPC):
***A61K 47/32*** (2006.01) ***A61K 9/48*** (2006.01)
***A61K 47/02*** (2006.01) ***A61K 47/04*** (2006.01)

(52) Cooperative Patent Classification (CPC):
**A61K 9/4816; A61K 9/4891; A61K 47/02; A61K 47/32**

(86) International application number:
**PCT/JP2019/038838**

(87) International publication number:
**WO 2020/071395 (09.04.2020 Gazette 2020/15)**

# (54) IMPROVED-STRENGTH HARD CAPSULE AND PRODUCTION METHOD FOR SAME

HARTKAPSEL MIT VERBESSERTER FESTIGKEIT UND HERSTELLUNGSVERFAHREN DAFÜR

GÉLULE DURE À RÉSISTANCE AMÉLIORÉE ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2018 JP 2018187610**

(43) Date of publication of application:
**11.08.2021 Bulletin 2021/32**

(73) Proprietor: **Qualicaps Co., Ltd.**
**Nara 639-1032 (JP)**

(72) Inventors:
- **ISHIKAWA, Tatsuya**
**Yamatokoriyama-shi, Nara 639-1032 (JP)**
- **HONDA, Mamoru**
**Yamatokoriyama-shi, Nara 639-1032 (JP)**

(74) Representative: **Müller-Boré & Partner Patentanwälte PartG mbB**
**Friedenheimer Brücke 21**
**80639 München (DE)**

(56) References cited:
**EP-A1- 3 862 021**
**WO-A1-2018/008660**
**WO-A1-2018/008660**
**WO-A1-2018/043661**
**CN-A- 104 224 746**
**JP-A- H06 179 618**
**JP-A- H06 179 618**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

[Technical Field]

The present invention relates to a hard capsule with improved strength, and a method for producing the hard capsule.

[Background Art]

Hard capsules have long been used as means for the preparation of oral formulations and are highly convenient in being able to encapsulate a wide variety of contents in the simplest manner possible and deliver the contents to the users.

The handleability in encapsulating a content into a hard capsule or filling a hard capsule with a content encapsulated therein into an external packaging material is referred to as runnability. When hard capsules are handled in a high-speed filling machine, local adsorption or squeezing may occur and generate local stress that causes deformation of the hard capsules. The degree of deformation depends on the elastic modulus of the capsule film within the range of its elastic deformation. Also, as the toughness of the film is higher, the capsules are less likely to crack even when deformed and can be stably handled at a higher speed, in other words, have better runnability. Also, when excessive local stress is instantaneously exerted on a hard capsule, the capsule may undergo excessive local deformation and crack. It can also be said that capsules have better runnability as they are less brittle. Strong hard capsules that have a property of having a high elastic modulus and being less likely to crack have a low risk of breaking and causing the content to leak or splatter during transportation or when the user touches them. Such handleability and convenience are advantages of hard capsules.

There are restrictions from the standpoint of safety on polymeric materials usable for pharmaceutical products or encapsulated food compositions, and it is impossible to take measures to increase the degree of crosslinking or the reactivity that induces crosslinking in order to improve the elastic modulus or strength such as crack resistance. However, crack resistance can be improved relatively easily independently of the elastic modulus without changing the structure of the polymeric material used as a primary component by controlling only the molecular weight, which has a small influence on the safety, in general, by increasing the molecular weight to enhance the entanglement of the main chains. On the other hand, because the elastic modulus of these polymeric materials hardly depends on the molecular weight once the basic skeleton and the proportion of substituents in the polymeric material for the capsule film are determined, it is difficult to improve the elastic modulus by controlling the molecular weight.

Also, mixtures of different types of polymeric materials are often not suitable as a hard capsule material because of their poor compatibility or poor moldability into capsules.

On the other hand, it may be possible to add some additives, preferably highly safe additives with a relatively low molecular weight that have been approved as pharmaceutical or food additives, but a material that can significantly improve the strength of a capsule film has not been known.

In the first place, a material that cannot form a flat and continuous film with a thickness of about 100 μm, which is suitable for a hard capsule, is not suitable as a primary component of a hard capsule film material. Also, when a large amount of commonly-used inorganic filler is added, it is difficult to obtain a flat film with a uniform thickness of about 100 μm. Naturally, safety concerns also remain.

Because it is necessary to select a primary component for a capsule film and improve its strength on a major premise of selecting a suitable combination of a polymeric material and additives for a hard capsule film as described above, it is apparent that simply adopting a strength improving method for a common polymeric material is totally insufficient.

Because hard capsules composed primarily of a polyvinyl alcohol soften under an environment in which the relative humidity at 25°C is higher than about 50% and embrittle at a relative humidity of about 20% or less, the strength of the capsule film must be improved so that the hard capsules can maintain a certain degree of strength under such environments. In Patent Document 1, a method for improving the hardness of a hard capsule composed primarily of a polyvinyl alcohol by adding a starch decomposition product, KUNIPIA-F, kaolin or talc to a capsule film is described.

[Related Art Document]

[Patent Document]

[Patent Document 1] WO2018/008660

[Summary of the Invention]

[Technical Problem]

When a hard capsule is used as an inhalation formulation capsule, a single dose of a drug is encapsulated in the hard capsule and the capsule is pierced with a small pin to enable inhalation of the drug inside at an appropriate flow rate. In this case, the capsule film does not have to be readily soluble but it is undesirable for the capsule film to undergo excessive deformation when pressed with a pin or extension of cracks or flaws from the periphery of the hole. This is because there is a possibility that broken pieces of the capsule film may be contained into the inhalation formulation inside the capsule or a stable amount of the drug may not be released. Thus, in order to form a small hole with a clear outline, the capsule film is required to have not only appropriate hardness but also appropriate strength when pierced with a pin.

The present invention aims to improve the strength of a capsule film for a hard capsule in order to achieve better runnability and the strength suitable for the above object.

[Solution to Problem]

The present inventors conducted intensive studies, and found that the addition of silica particles with an average particle size of 0.01 μm or more and 10 μm or less to a hard capsule film primarily composed of a polyvinyl alcohol can improve the strength of the capsule film for a hard capsule.

The present invention was made based on the above findings and includes the following embodiments.

Embodiment 1. A hard capsule, comprising a film containing a base of polyvinyl alcohols and silica particles with an average particle size of 0.01 μm or more and 10 μm or less, wherein the silica particles are contained in film components of the hard capsule in an amount of 3% by mass or more and 45% by mass or less based on the total of the film components excluding moisture, which is taken as 100% by mass, and wherein the polyvinyl alcohols are partially saponified polyvinyl alcohols with a degree of saponification in the range of 78% to 95%.

Embodiment 2. A hard capsule preparation liquid comprising a base of polyvinyl alcohols, silica particles with an average particle size of 0.01 μm or more and 10 μm or less, and a solvent, wherein the silica particles are contained in an amount of 3% by mass or more and 45% by mass or less based on the total solid content of the hard capsule preparation liquid excluding the solvent which is taken as 100% by mass, and wherein the polyvinyl alcohols are partially saponified polyvinyl alcohols with a degree of saponification in the range of 78% to 95%.

Embodiment 3. A method for preparing a hard capsule, comprising the step of preparing a hard capsule using a hard capsule preparation liquid according to embodiment 2.

[Advantageous Effects of Invention]

According to the present invention, it is possible to provide a polyvinyl alcohol hard capsule with improved strength, and a method for preparing the hard capsule.

[Brief Description of Drawings]

FIG. 1 shows an example of a typical tensile stress-strain curve in a tensile test, and an analysis example of elastic modulus (Young's modulus) and elongation at break. The elastic modulus is an inclination in an elastic region, and the elongation at break is the strain (%) at which the test piece breaks.

[Description of Embodiments]

1. Description of terms

(1) Hard capsule materials

First, the terms for use in the present specification, claims etc. are described. The terms used in the present invention comply with the description in this section unless otherwise stated.

In the present invention, a "hard capsule" is a type of capsule that is prepared by first producing a capsule film and then filling the produced capsule film with a content. Usually, a hard capsule consists of a cap portion and a body portion, and is also referred to as "two-piece capsule." The "hard capsule" of the present invention does not include a soft capsule produced by filling a content between two films and bonding the films to each other, a seamless capsule produced by dropping a content together with a film solution into a coagulating liquid, and a microcapsule prepared by

incorporating therein an active ingredient by precipitation or emulsification of a base material.

In the present invention, a "base" refers to a primary component for forming a hard capsule film. As the base, a chemically stable polymeric material which can be formed into a film (suitable for film formation) having appropriate strength after drying and which is hydrophilic and easily dissolved in the digestive system is preferred. Because the base is required to have safety and stability suitable for pharmaceutical products and food compositions, highly reactive or highly crosslinkable materials are not preferred. Polyvinyl alcohols (PVAs) can be used as a hydrophilic polymer in the present invention.

PVAs are polymerization products obtained by saponification of polyvinyl acetate. Usually, there are completely saponified products having a degree of saponification of 97 mol% or more and represented by the following formula (1) and partially saponified products having a degree of saponification of 78 to 96 mol% and represented by the following formula (2). In the present invention, both of the above-mentioned completely saponified products and partially saponified products can be used. A partially saponified product having a degree of saponification of 78 to 90%, in particular about 87 to 90%, is preferably used although there is no particular limitation.

[Chemical formula 1]

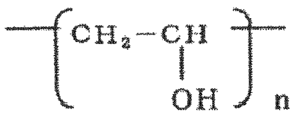

(1)

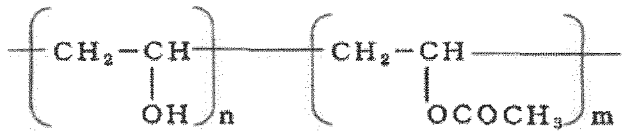

(2)

(wherein n and m each represent an arbitrary integer).

The number-average degree of polymerization (n) of the PVAs is not particularly limited as long as it is within a range in which a film forming ability can be exhibited, and is usually 400 to 3300, particularly preferably about 400 to 2000. Although the number-average molecular weight of the PVAs calculated from the number-average degree of polymerization and the degree of saponification described above is about 18000 to about 175000, the number-average molecular weight is not particularly limited thereto.

Examples of the PVA copolymers include PVA copolymers obtained by copolymerization of PVAs as described above or derivatives thereof with polymerizable vinyl monomers. Examples of the derivatives of PVAs here include known PVA derivatives such as amine-modified PVAs, ethylene-modified PVAs and PVAs having a thiol group at a terminal thereof (terminal thiol-modified PVAs).

Examples of the polymerizable vinyl monomers include (1) acrylic acid, methacrylic acid, fumaric acid, maleic acid, and itaconic acid; (2) sodium salts, potassium salts, ammonium salts or alkylamine salts of the compounds described in above (1); (3) methyl methacrylate, methyl acrylate, ethyl methacrylate, ethyl acrylate, butyl methacrylate, butyl acrylate, isobutyl methacrylate, isobutyl acrylate, cyclohexyl methacrylate, cyclohexyl acrylate, 2-ethylhexyl methacrylate, 2-ethylhexyl acrylate, acrylonitrile, acrylamide, dimethylacrylamide, styrene, vinyl acetate, hydroxyethyl methacrylate, hydroxyethyl acrylate, esters of polyethylene glycol and methacrylic acid, esters of polyethylene glycol and acrylic acid, esters of polypropylene glycol and methacrylic acid, esters of polypropylene glycol and acrylic acid, N-vinylpyrrolidone, or acryloyl morpholine; and (4) compounds represented by the following formula:

[Chemical formula 2] $H_2C=C(R^1)\text{-}COOR^2$

(wherein $R^1$ represents a hydrogen atom or a methyl group, and $R^2$ represents a hydrogen atom or an alkyl group having 1 to 4 carbon atoms). Preferably, as the polymerizable vinyl monomers, at least one compound selected from the group consisting of the compounds (1) and (2) and at least one compound selected from the group consisting of the compounds (3) are used in combination. Particularly preferred is a combined use of acrylic acid or methacrylic acid and methyl methacrylate.

A preferred PVA copolymer (not claimed) is a macromolecular copolymer obtained by copolymerization of acrylic acid with methyl methacrylate using a partially saponified PVA as described above as a skeleton. More preferred is a PVA copolymer obtained by copolymerization of a partially saponified PVA having an average degree of polymerization of about 300 to 500 with polymerizable vinyl monomers as described above (in particular, acrylic acid and methyl methacrylate) at a mass ratio of about 6:4 to 9:1. Here, more preferably, acrylic acid and methyl methacrylate are used as the polymerizable vinyl monomers at a mass ratio of about 3:7 to 0.5:9.5 in copolymerization with a partially saponified

PVA. A particularly preferred PVA copolymer is a PVA copolymer obtained by copolymerization of a partially saponified PVA having an average degree of polymerization of 300 to 500, methyl methacrylate and acrylic acid at a ratio (mass ratio) of 60 to 90:7 to 38:0.5 to 12.

Examples of commercially available PVA copolymers include POVACOAT (trademark) series (Nissin Kasei Co., LTD.).

Examples in which a PVA or PVA copolymer is applied to a hard capsule include those described in WO02/17848, WO1999/046329, WO2009/125483, and U.S Patent No. 6967026.

In the present invention, a PVA and a PVA copolymer may be used in combination. The mixing ratio between a PVA and a PVA copolymer in the film is not particularly limited. A PVA and a PVA copolymer may be used in any ratio in the range of PVA:PVA copolymer = 100:0 to 0:100 (mass ratio), preferably 99.9:0.1 to 0.1:99.9.

In the present invention, a "strength improver" refers to a component capable of improving the strength of a capsule film after preparation. The "strength" is intended to mean a parameter of a capsule film that can be evaluated by a method described later and is different from the hardness of a film that is represented by an elastic modulus and/or a toughness (calculated from the stress and elongation at break), for example, of a capsule film. Preferably, the "strength" is intended to mean elasticity (Young's modulus). One component or two or more components may be used as a strength improver. When two or more components are included in the strength improver, these two or more components may be mixed in advance before being dissolved in a solvent for a capsule preparation liquid, or may be individually dissolved in the solvent. Alternatively, the components dissolved separately in the solvent may be mixed. Preferably, the strength improver used in the present invention does not impair general properties required for use in pharmaceutical products or food compositions, such as safety, chemical stability (avoidance of reaction with the content), storage stability (change with the passage of time), light-shielding property, low oxygen permeability, low water vapor permeability, low moisture content, and constant chargeability.

One example of the strength improver is silica. The strength improver is silica particles. More preferably, the silica particles are spherical or generally spherical. The silica particles have an average particle size of 0.01 μm or more, for example. More preferably, the average particle size is 0.02 μm or more. The silica particles have ar average particle size of 10 μm or less, for example. More preferably, the average particle size is 5 μm or less. More preferably, the average particle size is 1 μm or less. The average particle size of silica particles can be obtained by measuring the sizes of the particles with an electron microscope or Coulter counter or by a laser diffraction scattering method, for example, and calculating an average thereof.

Examples of commercially available silica particles include AEROSIL series (manufactured by Nippon Aerosil Co., Ltd.), silica particles (Tokuyama Corporation Sunseal), and Sylosphere C-series (Fuji Silysia Chemical Ltd.).

The capsule film for a hard capsule of the present invention may contain, in addition to the base and the strength improver, a gelling agent, a gelling aid, a plasticizer, a lubricant, a sequestrant, a colorant, a light-shielding agent, residual moisture (also simply referred to as moisture), etc.

Examples of the gelling agent include carrageenan, tamarind seed polysaccharides, pectin, xanthan gum, locust bean gum, curdlan, gelatin, furcellaran, agar, and gellan gum. These can be used singly, or in any combination of two or more.

Among the above gelling agents, carrageenan, which has a high gel strength and can provide an excellent gelation effect in the presence of specific ions even when used in a small amount, is the optimum gelling agent. In general, three types of carrageenan are known: kappa-carrageenan, iota-carrageenan and lambda-carrageenan. In the present invention, kappa-carrageenan and iota-carrageenan, which have an ability to form a gel with relatively high strength, can be preferably used. Pectin can be classified into LM pectin and HM pectin according to the difference in the degree of esterification. Gellan gum can also be classified into acylated gellan gum (native gellan gum) and deacylated gellan gum according to the presence or absence of acylation. In the present invention, any of the above can be used regardless of the type.

A gelling aid can also be used according to the type of the gelling agent used. When carrageenan is used as a gelling agent, the following gelling aids can be used in combination with the gelling agent. For kappa-carrageenan, examples include compounds capable of donating one type or two or more types of ions selected from potassium ions, ammonium ions and calcium ions in water, such as potassium chloride, potassium phosphate, ammonium chloride, ammonium acetate and calcium chloride. For iota-carrageenan, examples include compounds capable of donating calcium ions in water, such as calcium chloride. When gellan gum is used as a gelling agent, examples of gelling aids that can be used in combination with the gelling agent include compounds capable of donating one type or two or more types of ions selected from sodium ions, potassium ions, calcium ions and magnesium ions in water, such as sodium chloride, potassium chloride, calcium chloride and magnesium sulfate. In addition, an organic acid or a water-soluble salt thereof, such as citric acid or sodium citrate, can also be used.

When a polyvinyl alcohol and a polyvinyl alcohol copolymer is used, the gelling agent that is preferably used in combination therewith is gellan gum. When a gelling agent is added to gellan gum, potassium chloride and/or calcium lactate can be preferably used as the gelling aid.

The plasticizer is not particularly limited as long as it can be used in pharmaceutical products or food compositions. Examples include dioctyl adipate, polyester adipate, epoxidized soybean oil, epoxyhexahydrophthalic acid diesters, kaolin, triethyl citrate, glycerin, glycerin fatty acid esters, sesame oil, dimethylpolysiloxane-silicon dioxide mixtures, D-sorbitol, medium-chain fatty acid triglyceride, corn starch-derived sugar alcohol liquid, triacetin, concentrated glycerin, castor oil, phytosterol, diethyl phthalate, dioctyl phthalate, dibutyl phthalate, butyl phthalyl butyl glycolate, propylene glycol, polyoxyethylene (105) polyoxypropylene (5) glycol, polysorbate 80, macrogol 1500, macrogol 400, macrogol 4000, macrogol 600, macrogol 6000, isopropyl myristate, cottonseed oil-soybean oil mixtures, glycerin monostearate and isopropyl linoleate. When a plasticizer is used, it is usually added in an amount in the range of 15% by mass or less, preferably 13% by mass or less, more preferably 11% by mass or less, still more preferably 8% by mass or less, based on the total of film components of the hard capsule excluding moisture, which is taken as 100% by mass.

Examples of the sequestrant include ethylenediaminetetraacetic acid, acetic acid, boric acid, citric acid, gluconic acid, lactic acid, phosphoric acid, tartaric acid, or salts thereof, methaphosphates, dihydroxyethylglycine, lecithin, β-cyclodextrin, or combinations thereof.

The lubricant is not particularly limited as long as it can be used in pharmaceutical products or food compositions. Examples include calcium stearate, magnesium stearate, sodium stearyl fumarate, carnauba wax, starch, sucrose fatty acid esters, light anhydrous silicic acid, macrogol, talc, and hydrogenated vegetable oils.

The colorant and light-shielding agent are not particularly limited as long as they can be used in pharmaceutical products or food compositions. Examples of the colorant include gambir tannin powder, turmeric extract, methylrosaniline chloride, yellow iron oxide, yellow ferric oxide, Opaspray K-1-24904, orange essence, brown iron oxide, carbon black, caramel, carmine, carotene liquid, β-carotene, photosensitizer 201, licorice extract, gold leaf, Sasa veitchii extract, black iron oxide, light anhydrous silicic acid, Daemonorops draco, zinc oxide, titanium oxide , iron sesquioxide, disazo yellow , Food Blue No. 1 and its aluminum lake, Food Blue No. 2 and its aluminum lake, Food Yellow No. 4 and its aluminum lake, Food Yellow No. 5 and its aluminum lake, Food Green No. 3 and its aluminum lake, Food Red No. 2 and its aluminum lake, Food Red No. 3 and its aluminum lake, Food Red No. 102 and its aluminum lake, Food Red No. 104 and its aluminum lake, Food Red No. 105 and its aluminum lake, Food Red No. 106 and its aluminum lake, sodium hydroxide, talc, sodium copper chlorophyllin, copper chlorophyll, hull-less barley green tea extract powder, hull-less barley green tea extract, phenol red, sodium fluorescein, d-borneol, malachite green, octyldodecyl myristate, methylene blue, medical carbon, riboflavin butyrate, riboflavin, green tea powder, ammonium manganese phosphate, sodium riboflavin phosphate, rose oil, turmeric color, chlorophyll, carminic acid color, Food Red No. 40 and its aluminum lake, water-soluble annatto, sodium iron chlorophyllin, dunaliella carotene, capsicum color, carrot carotene, potassium norbixin, sodium norbixin, palm oil carotene, beet red, grape pericarp color, black currant color, monascus color, safflower red color, safflower yellow color, marigold color, sodium riboflavin phosphate, madder color, alkanet color, aluminum, sweet potato carotene, shrimp color, krill color, orange color, cacao color, cacao carbon black, Japanese persimmon color, crayfish color, carob germ color, fish scale foil, silver, kusagi color, gardenia blue, gardenia red, gardenia yellow, kooroo color, chlorophyllin, kaoliang color, bone carbon black, bamboo grass color, shea nut color, shikon color, sandalwood red, vegetable carbon black, sappan color, spirulina color, onion color, tamarind color, corn color, tomato color, peanut color, phaffia color, pecan nut color, monascus yellow, powdered annatto, Haematococcus algae color, purple sweet potato color, purple corn color, purple yam color, vegetable oil soot color, lac color, rutin, enju extract, buckwheat whole-plant extract, logwood color, red cabbage color, red rice color, red radish color, adzuki bean color, Hydrangea leaves extract, sepia color, uguisukagura color, elderberry color, olive tea, cowberry color, gooseberry color, cranberry color, salmonberry color, strawberry color, dark sweet cherry color, cherry color, thimbleberry color, European dewberry color, pineapple juice, black huckleberry color, grape juice color, black currant color, blackberry color, plum color, blueberry color, berry juice, boysenberry color, whortleberry color, mulberry color, morello cherry color, raspberry color, red currant color, lemon juice, loganberry color, powdered chlorella, cocoa, saffron color, beefsteak plant color, chicory color, laver color, hibiscus color, malt extract, paprika, beet red juice, and carrot juice.

Examples of the light-shielding agent include titanium oxide, iron sesquioxide, yellow ferric oxide, black iron oxide, Food Blue No. 1 aluminum lake, Food Blue No. 2 aluminum lake, Food Yellow No. 4 aluminum lake, Food Yellow No. 5 aluminum lake, Food Green No. 3 aluminum lake, Food Red No. 2 aluminum lake, Food Red No. 3 aluminum lake, Food Red No. 102 aluminum lake, Food Red No. 104 aluminum lake, Food Red No. 105 aluminum lake, Food Red No. 106 aluminum lake, and Food Red No. 40 aluminum lake.

Titanium oxide may be added as a light-shielding agent to pharmaceutical hard capsules in order to prevent deterioration of the content caused by ultraviolet rays, for example.

It is usually preferred that the capsule film after preparation contains a few % of residual moisture. Usually, when molded capsules are subjected to a drying treatment at a temperature in the range of 30°C to 100°C, the moisture content of the capsules reaches a specified saturated residual moisture value corresponding to the solid content and composition of the capsules. Naturally, the time before the saturated moisture value is reached is shorter when the dry treatment is carried out at a higher temperature. The residual moisture changes almost reversibly although it also depends on the environmental humidity at the time of storage of the capsules. In other words, the saturated moisture value after

a sufficient dry treatment at 30 to 100°C converges to a certain value when the capsules are stored for several days at constant temperature and relative humidity. In the present invention, a saturated moisture value after storage for several days at room temperature and a relative humidity of 22%, 43% or 60% is used.

It is rather preferred that the capsule film contains a small amount of residual moisture in order to maintain crack resistance. The residual moisture, as saturated moisture value at room temperature and a relative humidity of 43%, is preferably at least 1% or more, more preferably 2% or more, still more preferably 3% or more based on the total mass of the capsule film. On the other hand, the residual moisture is preferably 8% or less, more preferably 6% or less because the residual moisture may react with the drug filled in the capsules if the residual moisture is too much when the capsules are stored for a long period of time.

The residual saturated moisture amount can be represented by the water content at a loss on drying and its measurement can be made as follows.

<Method for measuring water content in capsule film by loss-on-drying method>

A saturated aqueous solution of potassium carbonate is placed in a desiccator to create a constant-humidity atmosphere therein, and a sample (hard capsule or film) is placed in the desiccator. Then, the desiccator is sealed and subjected to humidity conditioning at 25°C for one week. In the presence of a saturated aqueous solution of potassium carbonate, an atmosphere with a relative humidity of about 43% can be created. After the mass (wet mass) of the sample after the humidity conditioning is measured, the sample is then dried by heating at 105°C for two hours, and the mass (dry mass) of the sample is measured again. From the difference between the mass before drying (wet mass) and the mass after drying (dry mass), the rate of the amount of moisture decreased during the drying by heating at 105°C for two hours (water content) is calculated according to the following equation.

[Mathematical Formula 1]

$$\text{Water content (\%)} = \frac{(\text{Wet mass of sample}) - (\text{Dry mass of sample})}{\text{Wet mass of sample}} \times 100$$

A layered clay mineral may be added to the capsule film. Examples of the "layered clay mineral" include phyllosilicates having a layered structure in which tetrahedral sheets and octahedral sheets are laminated. Examples of the phyllosilicates include lizardite, berthierine, amesite, cronstedtite, neopouite, kellyite, fraiponite, brindlleyite, kaolinite, dickite, nacrite, halloysite, odinite, talc, willemsite, kerolite, pimelite, pyrophyllite, ferripyrophyllite, saponite, hectorite, sauconite, stevensite, swinefordite, bentonite (composed primarily of montmorillonite), beidellite, nontronite, volkonskoite, vermiculite, biotite, phlogopite, annite, eastonite, siderophyllite, tetraferriannite, lepidolite, polylithionite, muscovite, celadonite, ferro-celadonite, ferro-aluminoceladonite, aluminoceladonite, tobelite, paragonite, illite, glauconite, brammallite, wonesite, clintonite, kinoshitalite, bityite, anandite, margarite, chlonichlore, chamosite, pennantite, nimite, baileychlore, donbassite, cookeite, sudoite, corrensite, hydrobiotite, aliettite, kulkeite, rectorite, tosudite, dozylite, lunijianlite, and saliotite. Preferred are bentonite, talc, kaolin etc. These layered clay minerals may be either natural minerals or synthetic minerals.

The surfaces of layered clay minerals are electrically charged because of isomorphic substitution or protonation or deprotonation of crystal end faces, and the charged state thereof is different in different layered clay minerals. To these layered clay minerals, substances such as inorganic and organic ions, polar molecules, and organic acids can adsorb.

In this disclosure, bentonite, for example, is a natural special colloidal clay, and is a colloidal hydrated aluminum silicate. Bentonite is said to be composed primarily of montmorillonite, which accounts for about 90% of bentonite, with the balance being accounted for by feldspar, calcium sulfate, beidellite, calcium carbonate, quartz, mica, manganese carbonate, etc.

Examples of commercially available bentonite include Veegum F, Veegum HV, and Veegum R (R.T. Vanderbilt C. Inc., USA); Kunipia-G and Kunipia-F (Kunimine Industries Co., Ltd.); Bentolite (Wilbur-Ellis); Bentonite TONEJIRUSHI (Kanben Mining Co., Ltd.); Bengel FW and Bengel (Nihon Yuukinendo Co., Ltd.); and Polargel NF (Volclay Japan Co., Ltd.).

In this disclosure, talc is a natural hydrated magnesium silicate, and is also referred to as talcum. Pure talc is $Mg_3Si_4O_{10}(OH)_2$ (molecular weight 379.27). Talc is composed primarily of $Mg_3Si_4O_{10}(OH)_2$, and is allowed to contain

chlorite (hydrated magnesium aluminum silicate), magnesite (magnesium carbonate), calcite (calcium carbonate), and dolomite (calcium magnesium carbonate) but does not contain asbestos.

The particle size of talc as measured by a laser diffraction-scattering method (JIS Z 8825:2013) is about 0.5 to 30 μm, preferably about 3.0 to 15.0 μm. The apparent density (JIS Z 2504:2012) is 0.12 to 0.40 g/$cm^3$, preferably 0.15 to 0.35 g/$cm^3$. The specific surface area as determined by a BET method (JIS Z 8830:2013) is about 2.5 to 40 $m^2$/g, preferably about 5 to 20 m2/g.

Examples of commercially available talc include Rose Talc, Micro Ace P-4, Micro Ace P-3, Micro Ace P-2, SG-95, and MS-KY (Nippon Talc Co., Ltd.); Talc Powder CT-250, Talc Powder CT-35 and Talc Powder EX-15 (Yamaguchi Mica Co., Ltd.); TALC JA-13R, TALC JA-24R, TALC JA-46R, TALC JA-68R, TALC JA-80R, TALC MMR, TALCSW-A, and TALC SW-Special (Asada Milling Co., Ltd.); IMP 1886L Talc BC (Ina Trading Co., Ltd.); and Luzenac Pharma (GSI Creos Corp.).

In this embodiment, kaolin corresponds to a natural hydrated aluminum silicate represented by $Al_2O_3 \cdot 2SiO_2/2H_2O$.

Examples of commercially available kaolin include 2747 Kaolin USP BC (Ina Trading Co., Ltd.), RF Amazonian White Clay (DKSH Japan K.K.), and White Clay and Red Clay (Matsumoto Trading Co., Ltd.).

## 2. Hard capsule

The film for a hard capsule according to this embodiment is composed of a film containing a base and a strength improver. The content of the base is obtained by subtracting the total mass % of the film components contained in the capsule film other than the base from the total of film components of the hard capsule excluding moisture, which is taken as 100% by mass.

The lower limit of the content of the strength improver is 3% by mass, of film components of the hard capsule based on the total of the film components excluding moisture, which is taken as 100% by mass. The upper limit of the content of the strength improver is 45% by mass, preferably 30% by mass, based on the total of film components of the hard capsule excluding moisture, which is taken as 100% by mass.

A more specific example of the composition of the hard capsule film including the strength improver is as follows: the content of the strength improver based on the total of film components of the hard capsule excluding moisture, which is taken as 100% by mass, is as described above with the balance being the base based on the total of film components of the hard capsule excluding moisture, which is taken as 100% by mass. Specifically, the base accounts for 45 to 99.9% by mass, preferably 55 to 99% by mass, more preferably 60 to 95% by mass, still more preferably 65 to 90% by mass, of the film components. When components other than the base and the strength improver are contained, a gelling agent may account for 0.025 to 2.5% by mass, preferably 0.05 to 2.3% by mass, more preferably 0.075 to 2% by mass, still more preferably 0.1 to 1.8% by mass of the film components. When a gelling aid such as potassium chloride is further contained, its content is in the range of 2.5% by mass or less, preferably 0.1 to 2.3% by mass, more preferably 0.15% to 2% by mass, still more preferably 0.2 to 1.8% by mass, of the film components. When the capsule film for a hard capsule according to this embodiment contains a plasticizer, its content is usually in the range of 15% by mass or less, preferably 13% by mass or less, more preferably 11% by mass or less, still more preferably 8% by mass or less, of the film components. Similarly, when a lubricant, a colorant, a light-shielding agent, a sequestrant, a flavoring agent etc. are contained, the content of each component can be set as appropriate in the range of 15% by mass or less, preferably 13% by mass or less, more preferably 11% by mass or less, still more preferably 8% by mass or less, of the film components.

Two or three strength improvers may be used in combination. When two or three strength improvers are used in combination, the lowest value among the lower limits of the above-mentioned contents of the strength improvers used in combination can be employed as the lower limit of the total content of the strength improvers contained in the hard capsule. When two or three strength improvers are used in combination, the highest value among the upper limits of the above-mentioned contents of the strength improvers used in combination can be employed as the upper limit of the total content of the strength improvers contained in the hard capsule.

When a layered clay mineral is added to the capsule film, the upper limit of the content of the layered clay mineral can be 5% by mass, preferably 3% by mass of film components based on the total of the film components excluding moisture, which is taken as 100% by mass. The lower limit of the content of the layered clay mineral can be 0.01% by mass, preferably 0.05% by mass of film components based on the total of the film components excluding moisture, which is taken as 100% by mass.

## 3. Hard capsule preparation liquid

The capsule preparation liquid for preparing a hard capsule according to this embodiment contains a solvent and the components as described above in section 2. The solvent is not particularly limited as long as it is an aqueous

solvent. The solvent is preferably water, ethanol, or a mixture thereof, more preferably water.

The concentrations of the above components contained in the hard capsule preparation liquid are not limited as long as the content of each component in the hard capsule after preparation can be equal to its content in the above hard capsule. In other words, the concentration of each component in the preparation liquid is not limited as long as the content of each component in the hard capsule after preparation based on the total solid content of the preparation liquid excluding the solvent, which is taken as 100% by mass, can be equal to its content in the above hard capsule. For example, the concentrations as described below can be used as the final concentrations in the capsule preparation liquid. The "final concentration" refers to the concentration in the finally obtained solution, that is, the concentration in the solution actually used to prepare a capsule.

The composition of the capsule preparation liquid is as follows. The base accounts for 9 to 20% by mass, preferably 11 to 19.5% by mass, more preferably 12 to 19% by mass, still more preferably 13 to 18% by mass; and the strength improver accounts for 0.02 to 10% by mass, preferably 0.2 to 6% by mass. When components other than the base and the strength improver are contained, a gelling agent may account for 0.005 to 0.5% by mass, preferably 0.01 to 0.45% by mass, more preferably 0.015 to 0.4% by mass. When a gelling aid is used, its concentration may be 0.5% by mass or less, 0.02 to 0.5% by mass, preferably 0.03 to 0.40% by mass, more preferably 0.04 to 0.35% by mass. When a lubricant, a colorant, a light-shielding agent, a sequestrant, a flavoring agent etc. are contained, the content of each component can be set as appropriate in the range of 0.5% by mass or less.

When two or three strength improvers are used in combination, the lower limit of the content of any of the above strength improvers can be employed as the lowest limit of the total content of the strength improvers contained in the hard capsule. Also, the upper limit of the content of any of the above strength improvers can be employed as the highest limit of the total content of the strength improvers contained in the hard capsule.

4. Method for preparing hard capsule

The method for preparing the capsule preparation liquid (dipping liquid) is not particularly limited. For example, there is a method for preparing a uniform capsule preparation liquid (dipping liquid) which includes dissolving a gelling agent or gelling aid, according to the needs, in purified water heated to about 70 to 100°C, followed by dissolving a polyvinyl alcohol and/or a polyvinyl alcohol copolymer in the solution.

The viscosity of the capsule preparation liquid is not particularly limited. Preferably, the capsule preparation liquid can be prepared to have a viscosity of 100 to 20000 mPa·s, preferably 300 to 10000 mPa s, under the temperature condition (temperature of dipping liquid) (30 to 80°C, preferably 40 to 60°C) that is employed when a capsule molding pin is dipped into the capsule preparation liquid. Usually, the capsule preparation liquid may have a solvent content of 60 to 90% by mass, preferably 70 to 85% by mass. The total content of film components of the hard capsule excluding the solvent in the capsule preparation liquid may be 10 to 40% by mass, preferably 15 to 30% by mass.

The viscosity defined in the present invention is a viscosity as measured with a B-type rotational viscometer at a predetermined temperature and at a rotational speed of 60 rpm with a measurement time of one minute using a No. 2 rotor for a viscosity of less than 500 mPa·s, a No. 3 rotor for a viscosity of 500 mPa·s or more and less than 2000 mPa·s and a No. 4 rotor for a viscosity of 2000 mPa·s or more.

The method for preparing (molding) a hard capsule is not particularly limited as long as it includes the step of preparing a capsule using a capsule preparation liquid according to the present invention. A hard capsule is generally obtained with desired capsule shape and thickness by dipping a mold pin serving as a mold for a capsule into an aqueous solution of a capsule film-forming material and curing and drying the film adhering to the mold pin when it is pulled up from the solution (dipping method). Specifically, the method for preparing a hard capsule includes a provision step of preparing a capsule preparation liquid by the above method or by purchasing a capsule preparation liquid, for example, and a preparation step of dipping a capsule molding pin into the capsule preparation liquid and then pulling up the capsule molding pin from the capsule preparation liquid to allow the solution adhering to the capsule molding pin to gelate followed by drying the gelated film at 20 to 80°C. In some cases, it is possible to achieve molding by cooling to increase the viscosity of the solution and drying it without the gelation step.

More specifically, the hard capsule used in the present invention can be produced through the following molding steps:

(1) a step of dipping a capsule molding pin into a capsule preparation liquid (dipping liquid) containing a polyvinyl alcohol and/or a polyvinyl alcohol copolymer (and a gelling agent and/or a gelling aid, when necessary) (dipping step),
(2) a step of pulling up the capsule molding pin from the capsule preparation liquid (dipping liquid) to allow the capsule preparation liquid adhering to an outer surface of the pin to gelate (gelation step),
(3) a step of drying the gelated capsule film (gelated film) formed to cover an outer surface of the capsule molding pin (drying step), and
(4) a step of releasing the dried capsule film (film) from the capsule molding pin (releasing step).

When necessary, the step (4) above may be followed by the following heating step:
(5) a step of subjecting the gelated capsule film (gelated film) to a heating treatment at 30 to 150°C after the gelation step (2) and before, after or simultaneously with the drying step (3) or after the releasing step (4) (heating step).

When a solution containing a gelling agent, such as carrageenan, is used as a capsule preparation liquid (dipping liquid), the above gelation step (2) may be performed by utilizing the fact that the solution gelates at a temperature of 50°C or lower, i.e., by setting the temperature around the capsule production apparatus to typically 35°C or lower, preferably 30°C or lower, preferably room temperature or lower to allow the capsule preparation liquid adhering to an outer surface of the capsule molding pin to cool (cold gelation method). Specifically, in the dipping step (1), the capsule molding pin, which has been adjusted to 10 to 30°C, preferably 13 to 28°C, more preferably 15 to 25°C depending on the liquid temperature of the capsule preparation liquid, is dipped into the capsule preparation liquid (dipping liquid), which has been adjusted to a constant temperature of 35 to 60°C, preferably 40 to 60°C. Then, in the gelation step (2), the capsule molding pin is pulled up from the capsule preparation liquid (dipping liquid) to allow the capsule preparation liquid adhering to an outer surface of the pin to gelate.

The drying step (3) can be performed at room temperature. Usually, the drying step (3) is performed at 80 to 150°C. The releasing step (4) is performed by removing a dried capsule film formed on a surface of the capsule molding pin from the capsule molding pin.

The optional heating step (5) can be performed after the gelation step (2), that is, after the capsule preparation liquid has been gelated (solidified). The heating treatment may be performed at any time after the gelation step (2), and may be performed before, after or simultaneously with the heating step (3), or after the releasing step (4). Preferably, after the gelation step (2), the gelated capsule film is subjected to a drying step at room temperature and a heating treatment is performed when the capsule film is dried or half-dried. The heating temperature is not particularly limited as long as it is in the range of 30 to 150°C, preferably 40 to 100°C, more preferably 50 to 80°C. The heating treatment can usually be performed by blowing air at 30 to 150°C.

The capsule films prepared in this way are cut to a predetermined length and can be provided as hard capsules with the paired body portion and cap portion fitted to each other or not.

Hard capsules usually have a film thickness in the range of 50 to 200 μm. In particular, currently commercially available capsules have a sidewall portion usually having a thickness of 70 to 150 μm, more preferably 80 to 120 μm. There are No. 00, No. 0, No. 1, No. 2, No. 3, No. 4, No. 5 and so on as the sizes of hard capsules. In the present invention, a hard capsule of any size can be used.

A capsule film can also be obtained by a solidification method that relies only on moisture evaporation from the capsule preparation liquid and drying without involving a gelation phenomenon.

## 5. Filling hard capsule with content and use thereof

The method for filling the hard capsule with a content is not particularly limited.

The hard capsule can be filled with a content with a known capsule-filling machine disclosed, for example, in JP2007-144014A or JP2000-226097A, such as a full-automatic capsule-filling machine (model name: LIQFIL super 80/150, manufactured by Qualicaps Co., Ltd.), and a capsule-filing and sealing machine (model name: LIQFIL super FS, manufactured by Qualicaps Co., Ltd.).

In the above filling method, temporal joining and permanent joining of hard capsules are ensured by a lock mechanism as disclosed in U.S. Patent No. 3508678 , U.S. Patent No. 3823843 , U.S. Patent No. 4040536 , U.S. Patent No. 4822618 , U.S. Patent No. 5769267, etc. The hardness of the hard capsule is also important to stably maintain such a lock mechanism.

In order to prevent malicious opening and entry of foreign matters, and to ensure the prevention of leakage of filled liquid, the capsule fitting portion may be sealed with the band seal as disclosed in JP2005-187412A or JP2009-504630A for more secure seal in addition to the above lock mechanism, which is achieved by rubbing the cap and body together.

The use of the hard capsule of the present invention is not particularly limited. Preferred examples of uses include oral formulations and inhalation formulations.

It is desirable that oral formulations are dissolved promptly in the stomach or intestine. In order to allow the capsule film to dissolve and release a drug in the intestine, an enteric capsule may be formed by applying a coating of an enteric base to the surface of the capsule film. An enteric capsule may also be formed by fabricating a capsule film itself exclusively or partially using an enteric base. The enteric capsule is not particularly limited as long as it has a property of not being dissolved in the stomach, but being dissolved in the intestine. For example, an enteric capsule refers to a capsule that is hardly dissolved in a dilute hydrochloric acid solution with a pH of 1.2 (Japanese Pharmacopoeia, first fluid) for more than 2 hours and is dissolved in a buffer solution with a pH of 6.8 (Japanese Pharmacopoeia, second fluid).

Further, it is possible to enable a drug to be released from the hard capsule in a sustained manner. For gradual

dissolution of a drug, the surface of the capsule film may be coated with a sustained-release film.

For inhalation formulations, a hard capsule in which a single dose of a drug has been encapsulated is loaded into a device, such as those disclosed in U.S. Patent No. 4069819, U.S. Patent No. 4210140, U.S. Patent No. 7669596, and U.S. Patent No. 2010-0300440A. The capsule is pierced with a small pin, or broken to enable inhalation of the drug inside at an appropriate flow rate.

The content encapsulated in the hard capsule is not particularly limited. Examples include, but are not limited to, pharmaceutical products, quasi-pharmaceutical products, cosmetics, and foods for humans and animals.

The form of the content is also not particularly limited. For example, the content may be in the form of a liquid, gel, powder, granules, tablets, pellets, or a mixture thereof (hybrid state).

Examples of the content that can be encapsulated in the hard capsule formulations include fillings such as common foods, health-promoting foods (foods with functional claims, foods with nutrient function claims, foods for specified health use), quasi-pharmaceutical products, and pharmaceutical product. Examples of the fillings include components derived from plants (including green unicellular alga) (e.g., raw plants, partially or fully dried plants, processed plant products and plant extracts), microorganisms (e.g., bacteria, yeasts and Euglena) or components derived from the microorganisms (e.g., raw microorganisms, partially or fully dried microorganisms, processed microorganism products, and microorganism extracts), and active ingredients such as nutritional fortification healthcare agents, antipyretic, analgesic, and anti-inflammatory agents, psychotropic agents, anxiolytic agents, antidepressants, hypnosedatives, anticonvulsive agents, central nervous system agents, brain metabolism-improving agents, brain circulation-improving agents, antiepileptic agents, sympathomimetic stimulants, gastrointestinal agents, antacids, anti-ulcer agents, antitussive and expectorant agents, antiemetic agents, anapnoics, bronchodilators, anti-allergic agents, agents for dental and oral use, antihistamines, cardiotonic agents, agents for arrhythmia, diuretic agents, hypotensive agents, vasoconstrictive agents, coronary vasodilators, peripheral vasodilators, agents for hyperlipidemia, cholagogues, antibiotics, chemotherapeutic agents, agents for diabetes, agents for osteoporosis, antirheumatic agents, skeletal muscle relaxants, spasmolytic agents, hormonal agents, alkaloidal narcotics, sulfa drugs, arthrifuges, anticoagulant agents, and antineoplastic agents or compositions containing the active ingredients. Such fillings are not particularly limited, and can be selected from a wide variety of known fillings. These components may be used singly or as a combined drug with other components. The fillings may be in any form such as solid, powder, granules, ground products, liquid, or gel. Also, these components are filled in a fixed known amount as appropriate based on the condition, age and so on of the administration target.

Examples of the nutritional fortification healthcare agents include vitamins such as vitamin A, vitamin D, vitamin E (e.g., d-$\alpha$-tocopherol acetate), vitamin B1 (e.g., dibenzoyl thiamine, fursultiamine hydrochloride), vitamin B2 (e.g., riboflavin butyrate), vitamin B6 (e.g., pyridoxine hydrochloride), vitamin C (e.g., ascorbic acid, sodium L-ascorbate), and vitamin B12 (e.g., hydroxocobalamin acetate, cyanocobalamin); minerals such as calcium, magnesium and iron; proteins; amino acids; oligosaccharides; and crude drugs.

Examples of the antipyretic, analgesic, and anti-inflammatory agents include, but are not limited to, aspirin, acetaminophen, ethenzamide, ibuprofen, diphenhydramine hydrochloride, chlorpheniramine dl-maleate, dihydrocodeine phosphate, noscapine, methylephedrine hydrochloride, phenylpropanolamine hydrochloride, caffeine, caffeine anhydride, serrapeptase, lysozyme chloride, tolfenamic acid, mefenamic acid, diclofenac sodium, flufenamic acid, salicylamide, aminopyrine, ketoprofen, indomethacin, bucolome, and pentazocine.

Examples of the common foods and health-promoting foods (foods with functional claims, foods with nutrient function claims, foods for specified health use) that can be encapsulated in the hard capsule according to this disclosure include, but are not limited to, fucoidan, heme iron, polyphenols, peptides and amino acids (e.g., royal jelly, ornithine, citrulline, aminolevulinic acid, black vinegar, or hydrophobic amino acids such as methionine, valine, leucine and isoleucine) , proteins (e.g., milk proteins such as lactoferrin, collagen and placenta), glycoproteins, enzyme-fermented foods (e.g., nattokinase), coenzymes (e.g., coenzyme Q10), vitamins (e.g., $\beta$-carotene), minerals, raw microorganisms (Euglena, chlorella, yeasts, lactobacillus and bifidobacteria) , plant extracts (e.g., crude drugs and herbs, such as turmeric extract, carrot extract, Japanese plum extract, ginkgo leaf extract, blueberry extract and Rubus suavissimus extract), and natural organic substances such as propolis, or any combination thereof.

## 6. Evaluation of strength

When the strength of a hard capsule film (Young's modulus, and/or elongation at break) is evaluated, it is important to compare test films having the same thickness. Thus, the strength of a film, which depends on the component composition of the hard capsule, can be evaluated using a cast film fabricated by a casting method using a preparation liquid having the same component composition as the component composition of the hard capsule preparation liquid.

The cast film is fabricated by placing a metal applicator on a surface of a glass or PET film held at room temperature, casting a preparation liquid at 50°C to 60°C and moving the metal applicator at a constant speed to form a uniform wet film that will have a thickness of 100 $\mu$m after drying. After that, the film is dried at a temperature of 80°C for about two hours. In order to obtain a film with a uniform thickness of 100 $\mu$m, applicators having gaps ranging from

0.4 mm to 1.5 mm may be used appropriately.

The fabricated film can be subjected to a tensile test using, for example, a compact tabletop tester (EZ-LX from Shimadzu Corporation) after being cut into a dumbbell shape of 5 mm $\times$ 75 mm (specified in JIS K-7161-2-1BA), for example. Specifically, both ends of the film are set on a holder (gap length 60 mm), and the film is pulled at a tension rate of 10 mm/min to obtain the elongation of the film and a curve showing the relation between the stress (tensile stress) that occurs in the film and the strain. FIG. 1 shows a typical tensile stress-strain curve. An elastic modulus, which is an indicator of hardness, can be obtained from the inclination in the elastic region under low stress in the graph, and a toughness ($MJ/m^3$), which is an indicator of crack resistance, can be obtained from the integral value of the tensile stress-strain curve in the range before the sample reaches the break point (Aqueous Polymeric Coating For Pharmaceutical Dosage Forms, 4th edition, CRC Press, 2017, Chapter 4).

It is desired that the above-mentioned strength is maintained in an environment under normal use conditions (at a temperature of about 5 to 30°C and a relative humidity of about 20 to 60%). Thus, in the present invention, after the fabricated film is subjected to humidity conditioning at 25°C and a relative humidity of 22% (low-humidity condition, saturated aqueous salt solution of potassium acetate is used) or a relative humidity of 60% (high-humidity condition, saturated aqueous salt solution of ammonium nitrate is used) for one week or longer, a tensile test is conducted in the same temperature and humidity environment as that for humidity conditioning to evaluate the mechanical strength.

The elastic modulus (Young's modulus), which is an indicator of hardness, is preferably 0.5 to 5 GPa. The toughness, which is an indicator of crack resistance evaluated by a tensile test, is preferably about 0.6 to 30 $MJ/m^3$. Usually, the hardness and crack resistance of the hard capsule film according to this disclosure are often in a trade-off relationship in these ranges. Coating films or soft capsule films are often softer and have a larger toughness. For example, a film having a toughness of more than 30 $MJ/m^3$ is too soft in many cases to be suitable as a self-supporting hard capsule film. On the other hand, when the toughness falls below 0.6 $MJ/m^3$, the capsule is prominently liable to crack easily even under normal handling conditions.

The moisture present in the capsule film in an amount of about a few % as described above may usually influence the strength, in particular cracking properties, as a plasticizer. Under use and storage conditions with a low relative humidity, the capsule film has a tendency to crack easily, that is, have a lower toughness when the moisture content is decreased to about 2 to 3%, for example. On the other hand, on the high humidity side, the capsule film tends to have an increased moisture content and have a lower elastic modulus. After all, the toughness is a problem on the low humidity side and the elastic modulus is a problem on the high humidity side. In this disclosure, in particular, moisture conditioning and a tensile test are performed in an environment with a relatively low relative humidity of 22% and a temperature of 25°C so that a film with a toughness of 0.6 to 30 $MJ/m^3$ can be obtained. Also, moisture conditioning and a tensile test are performed in an environment with a relatively high relative humidity of 60% and a temperature of 25°C so that a film having an elastic modulus of 0.5 to 5 GPa can be obtained. As a result, for the strength of the hard capsule according to this disclosure, an elastic modulus in the range of 0.5 to 5 GPa and a toughness of 0.6 to 30 $MJ/m^3$ can be obtained over the almost entire ranges of relative humidity and temperature in room conditions.

[Example]

The present invention is described more specifically below with reference to examples. However, the present invention should not be construed as limited to the examples.

<Example 1>

(1) Preparation of capsule composition liquid

144 g of deionized water was added to a 500 mL tall form beaker, and 35.7 g of a polyvinyl alcohol (EG-48P manufactured by Japan Synthetic Chemical Industry Co., Ltd.) as a base was added and dispersed under stirring with a mechanical stirrer. After the solution was heated to 85°C in a water bath, stirring was continued for another one hour to dissolve the polyvinyl alcohol. After 120 g of an aqueous silica slurry (WA302 manufactured by Fuji Silysia Chemical Ltd., silica concentration about 13% by mass, silica average particle size 0.2 $\mu$m) was added dropwise to the solution with a dropping funnel over about 40 minutes, stirring was continued for another one hour. The resultant solution was allowed to stand still and defoam overnight in an oven at 55°C and used as a capsule composition liquid. The capsule composition liquid was prepared such that the content of silica particles can be 30% by mass based on the total of film components excluding moisture when a film is formed.

(2) Preparation of film for evaluation

The capsule composition liquid prepared as described above was formed into a film such that the film would

have a dry film thickness of about 100 μm on a PET film using a box-type applicator, and the film was dried by allowing them to stand still in an oven at 80°C for two hours. The dried film was punched into a dumbbell shape (JIS K-7161-2 1-BA) using a punching machine and used as samples.

(3) Method for moisture conditioning of film for evaluation

The samples prepared as described above were allowed to stand still in a glass desiccator provided with a saturated aqueous salt solution, and moisture conditioning was performed by storing the desiccator in a constant temperature reservoir at 25°C for more than one week. A saturated aqueous salt solution of potassium acetate was used for a low humidity condition (25°C, 22% RH), and a saturated aqueous salt solution of ammonium nitrate was used for a high humidity condition (25°C, 60% RH).

(4) Evaluation of mechanical properties of film

The dumbbell-shaped test pieces (JIS K7161-2 1-BA) having subjected to moisture conditioning in a desiccator as described above were used for a tensile test at a tension rate of 10 mm/min and an interchuck distance of 59 mm. The device used was a universal tester EZ-LX manufactured by Shimadzu Corporation. By the tensile test, the elongation of the test pieces and the test force exerted thereon at that time were evaluated. A nominal stress σ, which is calculated by dividing the test force by the initial cross-sectional area, and a nominal strain, which is calculated by dividing the elongation of the test piece by its initial length, were obtained. The Young's modulus (MPa) was obtained from the initial inclination of the obtained stress-strain curve, and the toughness ($MJ/m^3$) of the film was obtained from the area under the stress-strain curve. The test pieces having subjected to moisture conditioning at 25°C and 60% RH were used for the evaluation of Young's modulus, and the test pieces having subjected to moisture conditioning at 25°C and 22% RH were used for the evaluation of toughness.

<Example 2>

Film samples were prepared and evaluated in the same manner as in Example 1 except that an aqueous silica slurry obtained by adding 25.5 g of AEROSIL 90G (manufactured by Nippon Aerosil Co., Ltd., silica average particle size 0.02 μm) to 229.6 g of deionized water and irradiating the mixture with ultrasonic waves for four hours was used. The content of the silica particles based on the total of film components excluding moisture was set to be equal to that in Example 1.

<Example 3>

258 g of deionized water was added to a 500 mL tall form beaker, and 12.6 g of silica particles (Sunseal SS10 manufactured by Tokuyama Corporation, average particle size 1 μm) was added. The mixture was subjected to an ultrasonic treatment for two hours. Under stirring with a mechanical stirrer, 29.4 g of a polyvinyl alcohol (EG-48P manufactured by Japan Synthetic Chemical Industry Co., Ltd) was added and dispersed. After the solution was heated to 85°C in a water bath, stirring was continued for another one hour to dissolve the polyvinyl alcohol. The resultant solution was allowed to stand still and defoam in an oven at 55°C and used as a capsule composition liquid. Except the above, film samples were prepared and evaluated in the same manner as in Example 1. The content of the silica particles based on the total of film components excluding moisture was set to be equal to that in Example 1.

<Example 4>

Film samples were prepared and evaluated in the same manner as in Example 3 except that Sylosphere C-1504 manufactured by Fuji Silysia Chemical Ltd. (average particle size 4 μm) , was used as silica particles. The content of the silica particles based on the total of film components excluding moisture was set to be equal to that in Example 1.

<Example 5>

Film samples were prepared and evaluated in the same manner as in Example 3 except that Sylosphere C-1510 manufactured by Fuji Silysia Chemical Ltd.(average particle size 10 μm) was used as silica particles. The content of the silica particles based on the total of film components excluding moisture was set to be equal to that in Example 1.

<Comparative Example 1>

Film samples were prepared and evaluated in the same manner as in Example 1 except that no silica slurry was added.

<Example 6>

Film samples were prepared and evaluated in the same manner as in Example 3 except that the amount of silica particles added was adjusted such that the content of silica particles based on the total of film components excluding moisture would be 15.8% by mass.

<Example 7>

Film samples were prepared and evaluated in the same manner as in Example 3 except that the amount of silica particles added was adjusted such that the content of silica particles based on the total of film components excluding moisture would be 42% by mass.

<Example 8>

Film samples were prepared and evaluated in the same manner as in Example 1 except that the amount of silica slurry added was adjusted such that the content of silica particles based on the total of film components excluding moisture would be 42% by mass.

<Example 9>

174 g of deionized water was added to a 500 mL tall form beaker, and 0.42 g of montmorillonite (Kunipia-F manufactured by Kunimine Industries Co., Ltd.) was added. The fluid dispersion was subjected to a homogenization treatment (10000 rpm, 30 minutes) using a homogenizer (manufactured by IKA, the generator used: S25N-25F). 28.2 g of a polyvinyl alcohol (EG-48P manufactured by Japan Synthetic Chemical Industry Co., Ltd) was added and dispersed under stirring with a mechanical stirrer. After the solution was heated to 85°C in a water bath, stirring was continued for another one hour to dissolve the polyvinyl alcohol. After 97 g of an aqueous silica slurry (WA302 manufactured by Fuji Silysia Chemical Ltd., silica concentration about 13% by mass, silica average particle size 0.2 μm) was added dropwise to the solution with a dropping funnel over about 40 minutes, stirring was continued for another one hour. The resultant solution was allowed to stand still and defoam overnight in an oven at 55°C and used as a capsule composition liquid. Except the above, film samples were prepared and evaluated in the same manner as in Example 1. The content of the silica particles based on the total of film components excluding moisture was set to be equal to that in Example 1. The content of montmorillonite based on the total of film components excluding moisture was adjusted to 1% by mass.

<Example 10>

Film samples were prepared and evaluated in the same manner as in Example 9 except that the content of montmorillonite based on the total of film components excluding moisture was adjusted to 2% by mass. The content of the silica particles based on the total of film components excluding moisture was set to be equal to that in Example 9.

Strength properties of capsule films

The film components and the results of evaluation in Examples 1 to 10 and Comparative Example 1 are summarized in Table 1.

[Table 1]

| | Base | Silica particles | | Montmorillonite | Young's modulus (MPa) at 25°C, 60% RH | Toughness (MJ/m$^3$) at 25°C, 22%RH |
|---|---|---|---|---|---|---|
| | | Average particle size (μm) | Content (% by mass) | Content (% by mass) | | |
| Ex. 1 | PVA | 0.2 | 30 | - | 1640 | 19 |
| Ex. 2 | PVA | 0.02 | 30 | - | 1580 | 9 |

(continued)

| | Base | Silica particles | | Montmorillonite | Young's modulus (MPa) at 25°C, 60% RH | Toughness (MJ/$m^3$) at 25°C, 22%RH |
|---|---|---|---|---|---|---|
| | | Average particle size (μm) | Content (% by mass) | Content (% by mass) | | |
| Ex. 3 | PVA | 1 | 30 | - | 890 | 10 |
| Ex. 4 | PVA | 4 | 30 | - | 940 | 1 |
| Ex. 5 | PVA | 10 | 30 | - | 780 | 0.6 |
| Comp. Ex. 1 | PVA | - | - | - | 380 | 104 |
| Ex. 6 | PVA | 1 | 15.8 | - | 500 | 18 |
| Ex. 7 | PVA | 1 | 42 | - | 1190 | 2 |
| Ex. 8 | PVA | 0.2 | 42 | - | 2050 | 2 |
| Ex. 9 | PVA | 0.2 | 30 | 1 | 2020 | 7 |
| Ex. 10 | PVA | 0.2 | 30 | 2 | 2380 | 4 |

Compared to Comparative Example 1, in which no silica particle was contained, the toughness was decreased but a Young's modulus that is twice as high as that in Comparative Example 1 was obtaines in Examples 1 to 5, in which silica partiles with various average particle sizes were added. From the standpoint of the ability to reduce a decrease in toughness, it was found that the silica particles preferably have an average particles sizes of 1μm or less.

In Examples 6 to 8, the content of silica particles was changed within the range of 15.8 to 42% by mass, but a Young's modulus higher than that in Comparative Example 1 was also obtained in this range.

In Examples 9 and 10, the addition of a layered clay mineral to the film of Example 1 was proved to be more effective in improving the Young's modulus.

## Claims

**1.** A hard capsule, comprising a film containing a base of polyvinyl alcohols and silica particles with an average particle size of 0.01 μm or more and 10 μm or less,

wherein the silica particles are contained in film components of the hard capsule in an amount of 3% by mass or more and 45% by mass or less based on the total of the film components excluding moisture, which is taken as 100% by mass, and
wherein the polyvinyl alcohols are partially saponified polyvinyl alcohols with a degree of saponification in the range of 78% to 95%.

**2.** The hard capsule according to Claim 1, wherein the average particle size of the silica particles is 0.02 μm or more and 1 μm or less.

**3.** The hard capsule according to Claim 1 or 2, wherein the film further contains a layered clay mineral.

**4.** The hard capsule according to Claim 3, wherein the layered clay mineral is a phyllosilicate.

**5.** The hard capsule according to Claim 4, wherein the phyllosilicate is bentonite.

**6.** A hard capsule preparation liquid comprising a base of polyvinyl alcohols, silica particles with an average particle size of 0.01 μm or more and 10 μm or less, and a solvent,

wherein the silica particles are contained in an amount of 3% by mass or more and 45% by mass or less based on the total solid content of the hard capsule preparation liquid excluding the solvent which is taken as 100% by mass, and

wherein the polyvinyl alcohols are partially saponified polyvinyl alcohols with a degree of saponification in the range of 78% to 95%.

7. The hard capsule preparation liquid according to Claim 6, wherein the average particle size of the silica particles is 0.02 μm or more and 1 μm or less.

8. The hard capsule preparation liquid according to Claim 6 or 7, further comprising a layered clay mineral.

9. The hard capsule preparation liquid according to Claim 8, wherein the layered clay mineral is a phyllosilicate.

10. The hard capsule preparation liquid according to Claim 9, wherein the phyllosilicate is bentonite.

11. A method for preparing a hard capsule, comprising the step of preparing a hard capsule using a hard capsule preparation liquid according to any one of claim 6 to 10.

12. The method for preparing a hard capsule according to Claim 11, wherein the method for preparing a hard capsule is for improving the hardness of a hard capsule.

**Patentansprüche**

1. Hartkapsel, umfassend eine Schicht, enthaltend eine Basis aus Polyvinylalkoholen und Siliziumdioxidteilchen mit einer durchschnittlichen Teilchengröße von 0,01 μm oder mehr und 10 μm oder weniger,

   wobei die Siliziumdioxidteilchen in den Schichtbestandteilen der Hartkapsel in einer Menge von 3 Massen-% oder mehr und 45 Massen-% oder weniger enthalten sind, bezogen auf die Gesamtheit der Schichtbestandteile ausgenommen Feuchtigkeit, welche als 100 Massen-% genommen wird, und
   wobei die Polyvinylalkohole teilweise verseifte Polyvinylalkohole mit einem Verseifungsgrad im Bereich von 78% bis 95% sind.

2. Hartkapsel nach Anspruch 1, wobei die durchschnittliche Teilchengröße der Siliziumdioxidteilchen 0,02 μm oder mehr und 1 μm oder weniger beträgt.

3. Hartkapsel nach Anspruch 1 oder 2, wobei die Schicht weiter ein geschichtetes Tonmineral enthält.

4. Hartkapsel nach Anspruch 3, wobei das geschichtete Tonmineral ein Phyllosilicat ist.

5. Hartkapsel nach Anspruch 4, wobei das Phyllosilicat Bentonit ist.

6. Hartkapselherstellungsflüssigkeit, umfassend eine Basis aus Polyvinylalkoholen, Siliziumdioxidteilchen mit einer durchschnittlichen Teilchengröße von 0,01 μm oder mehr und 10 μm oder weniger und ein Lösungsmittel,

   wobei die Siliziumdioxidteilchen in einer Menge von 3 Massen-% oder mehr und 45 Massen-% oder weniger enthalten sind, bezogen auf den gesamten Feststoffgehalt der Hartkapselherstellungsflüssigkeit ausgenommen das Lösungsmittel, welcher als 100 Massen-% genommen wird, und
   wobei die Polyvinylalkohole teilweise verseifte Polyvinylalkohole mit einem Verseifungsgrad im Bereich von 78% bis 95% sind.

7. Hartkapselherstellungsflüssigkeit nach Anspruch 6, wobei die durchschnittliche Teilchengröße der Siliziumdioxidteilchen 0,02 μm oder mehr und 1 μm oder weniger beträgt.

8. Hartkapselherstellungsflüssigkeit nach Anspruch 6 oder 7, weiter umfassend ein geschichtetes Tonmineral.

9. Hartkapselherstellungsflüssigkeit nach Anspruch 8, wobei das geschichtete Tonmineral ein Phyllosilicat ist.

10. Hartkapselherstellungsflüssigkeit nach Anspruch 9, wobei das Phyllosilicat Bentonit ist.

11. Verfahren zum Herstellen einer Hartkapsel, umfassend den Schritt des Herstellens einer Hartkapsel unter Verwendung einer Hartkapselherstellungsflüssigkeit nach einem der Ansprüche 6 bis 10.

12. Verfahren zum Herstellen einer Hartkapsel nach Anspruch 11, wobei das Verfahren zum Herstellen einer Hartkapsel zum Verbessern der Härte einer Hartkapsel ist.

**Revendications**

1. Gélule dure, comprenant un film contenant une base d'alcools polyvinyliques et des particules de silice d'une taille de particules moyenne de 0,01 μm ou plus et de 10 μm ou moins,

   dans laquelle les particules de silice sont contenues dans les composants de film de la gélule dure dans une proportion de 3 % en masse ou plus et de 45 % en masse ou moins par rapport au total des composants de film à l'exclusion de l'humidité, qui est considérée comme étant de 100 % en masse, et
   dans laquelle les alcools polyvinyliques sont des alcools polyvinyliques partiellement saponifiés dont le degré de saponification est compris entre 78 % et 95 %,

2. Gélule dure selon la revendication 1, dans laquelle la taille de particules moyenne de silice est de 0,02 μm ou plus et de 1 μm ou moins,

3. Gélule dure selon la revendication 1 ou 2, dans laquelle le film contient en outre un minéral argileux stratifié.

4. Gélule dure selon la revendication 3, dans laquelle le minéral argileux stratifié est un phyllosilicate.

5. Gélule dure selon la revendication 4, dans laquelle le phyllosilicate est la bentonite.

6. Liquide de préparation de gélule dure comprenant une base d'alcools polyvinyliques, des particules de silice d'une taille de particules moyenne de 0,01 μm ou plus et de 10 μm ou moins, et un solvant,

   dans lequel les particules de silice sont contenues dans une quantité de 3 % en masse ou plus et de 45 % en masse ou moins par rapport à la teneur totale en solides du liquide de préparation de gélule dure à l'exclusion du solvant qui est considéré comme étant de 100 % en masse, et
   dans lequel les alcools polyvinyliques sont des alcools polyvinyliques partiellement saponifiés dont le degré de saponification est compris entre 78 % et 95 %.

7. Liquide de préparation de gélule dure selon la revendication 6, dans lequel la taille de particules moyenne de silice est de 0,02 μm ou plus et de 1 μm ou moins,

8. Liquide de préparation de gélule dure selon la revendication 6 ou 7, comprenant en outre un minéral argileux stratifié.

9. Liquide de préparation de gélule dure selon la revendication 8, dans lequel le minéral argileux stratifié est un phyllosilicate.

10. Liquide de préparation de gélule dure selon la revendication 9, dans lequel le phyllosilicate est de la bentonite.

11. Procédé de préparation d'une gélule dure, comprenant l'étape de préparation d'une gélule dure à l'aide d'un liquide de préparation de gélule dure selon l'une quelconque des revendications 6 à 10.

12. Procédé de préparation d'une gélule dure selon la revendication 11, dans lequel le procédé de préparation d'une gélule dure est destiné à améliorer la dureté d'une gélule dure.

Fig.1

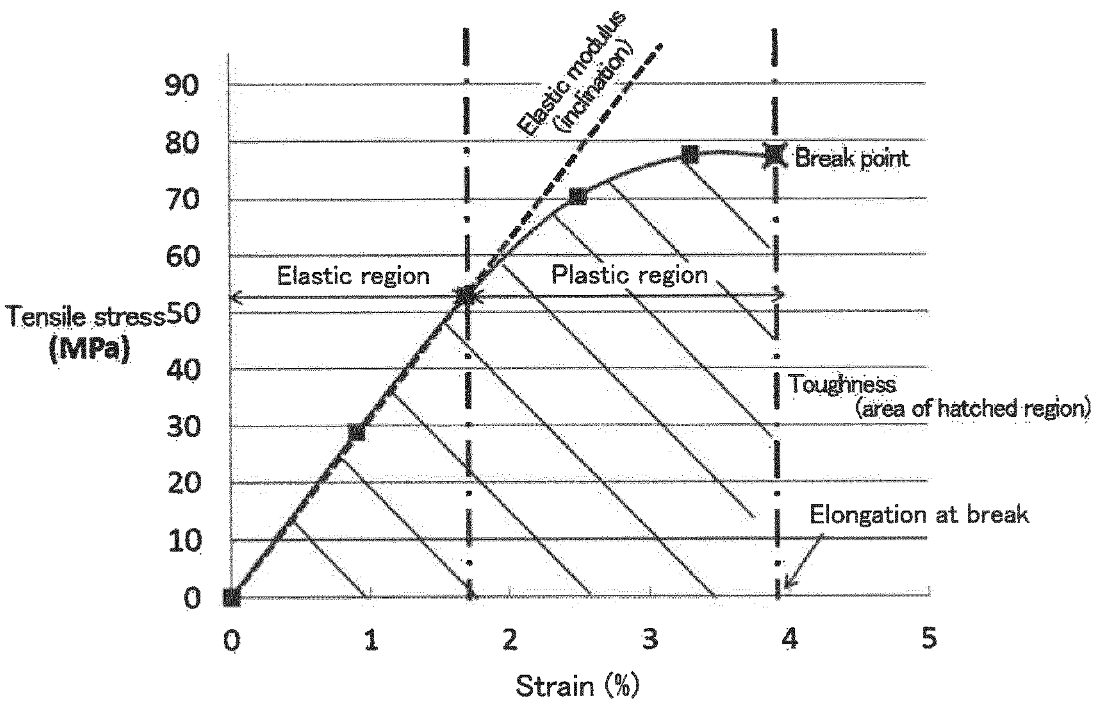
Elastic modulus
(inclination)
90
80
70
60
50
40
30
20
10
0
Tensile stress
(MPa)
Break point
Elastic region
Plastic region
Toughness
(area of hatched region)
Elongation at break
0
1
2
3
4
5
Strain (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018008660 A
- WO 0217848 A
- WO 1999046329 A
- WO 2009125483 A
- US 6967026 B
- JP 2007144014 A
- JP 2000226097 A
- US 3508678 A
- US 3823843 A
- US 4040536 A
- US 4822618 A
- US 5769267 A
- JP 2005187412 A
- JP 2009504630 A
- US 4069819 A
- US 4210140 A
- US 7669596 B
- US 20100300440 A


### Non-patent literature cited in the description


- Aqueous Polymeric Coating For Pharmaceutical Dosage Forms. CRC Press, 2017